# EUROPEAN PATENT SPECIFICATION

(11) **EP 0 514 085 B1**
(45) Date of publication and mention of the grant of the patent: **02.07.1997**
(21) Application number: 92304131.3
(22) Date of filing: 08.05.1992
(51) Int. Cl.: A61M 15/00, A61M 16/00

(54) **Aerosol inhalation device**
Aerosol-Inhalationsgerät
Inhalateur pour aerosol

(30) Priority: 14.05.1991 AU 6139/91
(43) Date of publication of application: 19.11.1992
(73) Proprietor: SCOTT DIBBEN PTY LIMITED, Newcastle New South Wales (AU)
(72) Inventor: Smith, Colin Marshall, Merewether, New South Wales 2291 (AU)
(74) Representative: Perkins, Sarah

(56) References cited:
- GB-A- 2 228 873
- US-A- 4 259 951
- US-A- 4 534 343

## Description

The following invention relates to an aerosol inhalation device particularly useful for the inhalation of aerosol drug particles by an asthmatic.

Medication inhalers of the above type are known. Such inhalers comprise an elongated aerosol holding chamber, through a remote end from a user of which, aerosol drug particles are received. At an end of the chamber proximal to the user, a diaphragm valve is often provided. Such a valve is adapted to open as the user inhales so as to allow passage therethrough of the particulate medication from the chamber. Upon the user's exhaling, the diaphragm valve closes to prevent passage of breath therethrough back into the chamber. Several breaths are often required for administration of the required drug dosage. It is a disadvantage of such devices that it can be quite difficult for the user to exhale, as in order to establish proper operation of the diaphragm valve, it is necessary to substantially inhibit the flow of exhaled breath.

Furthermore, most known devices of this type have substantial flow passage "dead zones" which result in wasted medication and general inefficiency.

Other devices comprise spring loaded valve mechanisms which are cumbersome and expensive to manufacture.

US Patent No. 4,534,343 discloses an aerosol inhalation device according to the preamble of present Claim 1 and is considered to represent the closest prior art. Further, it attempts to provide a double acting valve in an aerosol inhalation device by providing engagement of the inner portion of a diaphragm with an inner end 74 of a mouthpiece. This US patent shows a diaphragm 64 comprising a pair of cross slits. When the central region of the diaphragm 64 opens, a gap is formed between the adjacent triangular sections separated by the slits. That is, upon inhaling, the patient using the apparatus of this US patent would inhale air directly from the atmosphere through apertures 76.

GB-A-2 228 873 discloses an inhaling apparatus comprising apertures 52, whereby when a user inhales, air flows from atmosphere through the apertures 52 and thus is inhaled by the user.

US 4,259,951 discloses an inhaling device having a complicated two-way valve formed with a diaphragm having two large flaps 60 and a large number of small flaps 52 formed therein. The diaphragm is positioned between two facing surfaces with differently sized apertures formed therein such that the two large flaps will open in a first direction to allow air and vapour to pass through the diaphragm in that first direction, however the large flaps will not open in the opposite direction, thus preventing the passage of air and vapour in that direction. Furthermore the arrangement is such that the large number of small flaps will not open in the first direction but will in the opposite direction, thus providing the two-way valve.

It is the object of the present invention to overcome or substantially ameliorate at least one of the above disadvantages.

According to the present invention there is provided apparatus comprising:
a housing to co-operate with, attached to, or formed integrally with a chamber to define a major fluid passage comprising an upstream zone within the chamber and a downstream zone;
a major fluid exit provided in the downstream zone;
a back-flow fluid exit comprising an annular passage surrounding said major fluid passage; and
a diaphragm mounted within the housing and comprising a central region comprising at least two slits defining at least one flap interfacing the upstream and downstream zones and being openable to allow fluid flow therethrough from the upstream zone to the downstream zone and through the major fluid exit;
the apparatus being characterised by:
said annular passage comprising an annular sealing ledge; and
said diaphragm further comprising a continuous peripheral region across the back-flow fluid exit which is in selective fluid communication with the downstream zone, is closable by said peripheral region resting against said sealing ledge when the central region is opened, and is openable upon deflection of the peripheral region away from said sealing ledge when the central region is closed and when fluid pressure in the downstream zone is higher than ambient pressure so as to allow escape of fluid from the downstream zone to atmosphere via the back-flow fluid exit.

There is further disclosed herein in accordance with a preferred form of the invention a combination comprising the diaphragm and a chamber having formed thereon a grid extending across the major fluid passage adjacent to and supporting the diaphragm, which grid comprises passages extending therethrough.

In accordance with a further preferred form of the invention, there is further disclosed herein an aerosol inhalation device comprising an aerosol holding chamber having disposed at one end thereof a diaphragm valve mechanism in accordance with the present invention mentioned above.

A preferred form of the present invention will now be described by way of example with reference to the accompanying drawings, wherein:
Fig. 1 is a schematic cross-sectional elevational view of a diaphragm valve mechanism showing the diaphragm valve in two operational positions,
Fig. 2 is a schematic cross-sectional elevational view of an aerosol inhalation device comprising the diaphragm valve mechanism of Fig. 1,
Fig. 3 is a schematic end elevational view of a diaphragm valve,
Figs. 4(a) and 4(b) are schematic end elevational and side elevational views respectively of a diaphragm support structure forming part of the diaphragm valve mechanism depicted in Fig. 1,
Figs. 5(a) and 5(b) are schematic side elevational and end elevational views respectively of a housing forming part of the diaphragm valve mechanism depicted in Fig. 1,

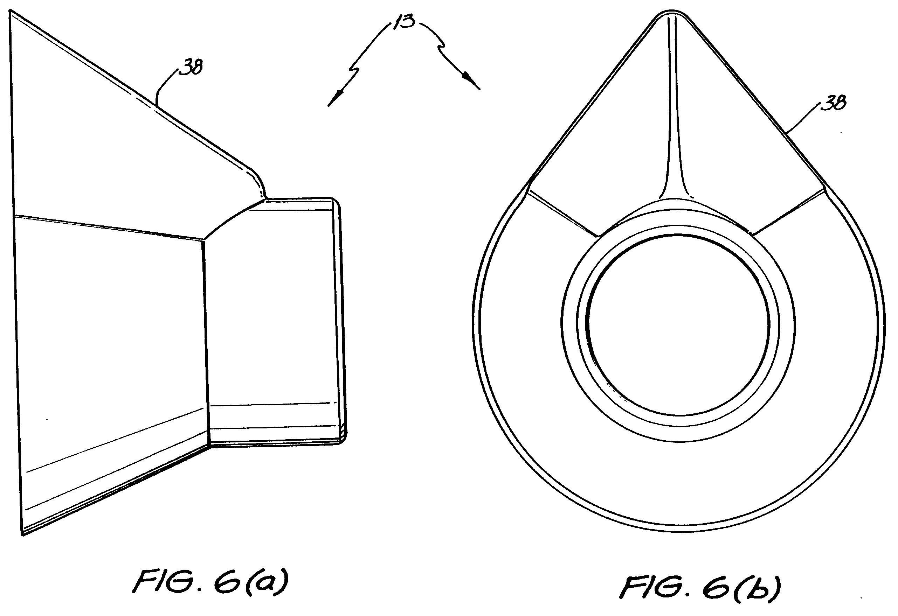

Figs. 6(a) and 6(b) are schematic side elevational and end elevational views respectively of a mask adapted to be attached to the aerosol inhalation device depicted in Fig. 2, and

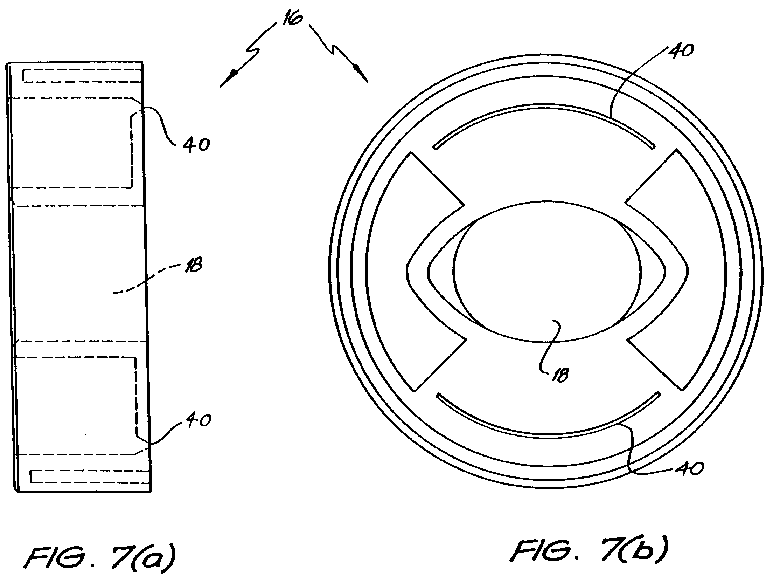

Figs. 7(a) and 7(b) are schematic side elevational and end elevational views respectively of a flexible end cap forming part of the aerosol inhalation device depicted in Fig. 2.

### DETAILED DESCRIPTION OF THE PREFERRED EMBODIMENT

In the accompanying drawings there is schematically depicted an aerosol inhalation device 1. Device 1 comprises an aerosol holding chamber 15 having attached to one end thereof (the end distal of a user when in use) a flexible cap 16. Flexible cap 16 is adapted to fit over the distal end of cylindrical shell 17 and comprises an aperture 18 through which a standard inhaler mouthpiece is sealingly inserted so as to dispense airborne medication into chamber 15. Flexible cap 16 further comprises a number of valve slits 40 which open inwardly towards chamber 15 when fluid pressure therein is lower than ambient pressure. The tapered form of each valve slits 40 prevents opening thereof when fluid pressure within chamber 15 is higher than ambient pressure.

As an alternative to the provision of valve slit 40, any form of valve mechanism may be provided in cap 16.

Aerosol holding chamber 15 in the preferred embodiment comprises a cylindrical shell 17 having mounted thereto or formed integrally at the proximal end thereof, a housing 23 surrounding a diaphragm valve mechanism 10.

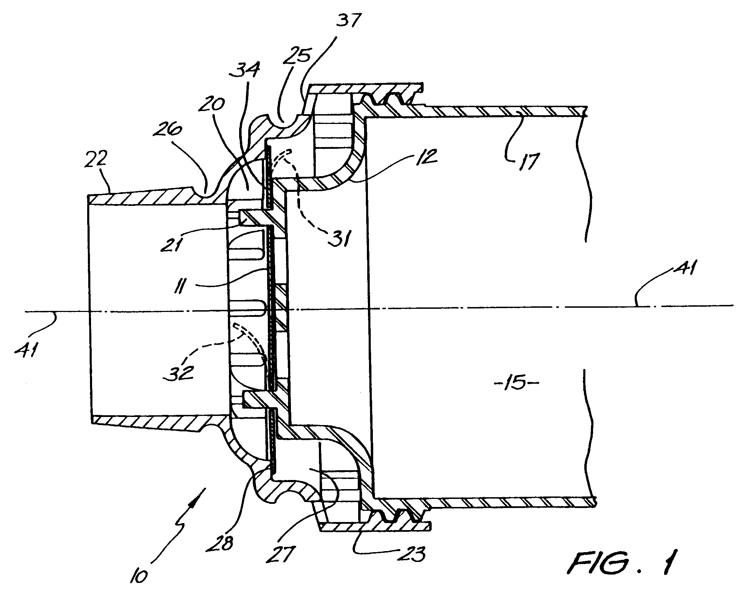

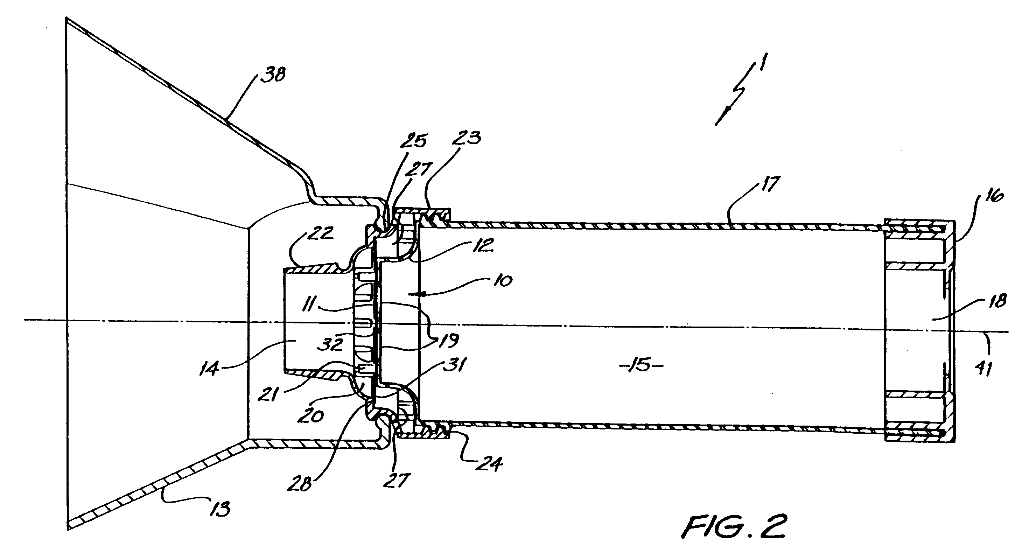

Diaphragm valve mechanism 10 comprises a diaphragm valve 11 supported by a supporting structure 12. Cylindrical shell 17, housing 23 and diaphragm valve support structure 12 are typically formed of a moulded plastics material. Attached to housing 23 and as shown in Figs. 1 and 2 via a large annular recess 25 is a face mask 13. Provision is made by way of a small annular recess 26 for fitting of a smaller child or infant sized face mask (not shown). Face mask 13 may be reversed and attached to annular recess 25 so as to locate mask 13 in a convenient storage position over and around cylindrical shell 17.

Face mask 13 is an optional feature as mouthpiece 22 is provided surrounding exit port 14 of housing 23.

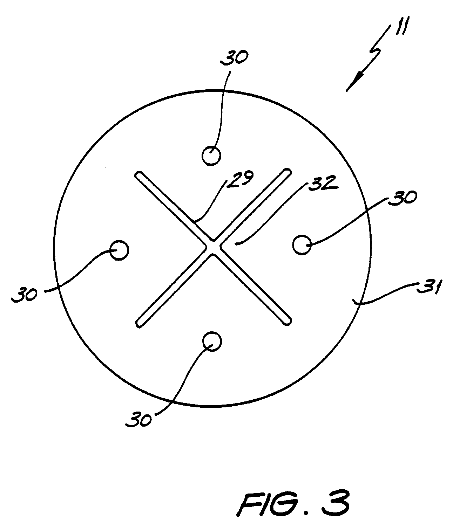

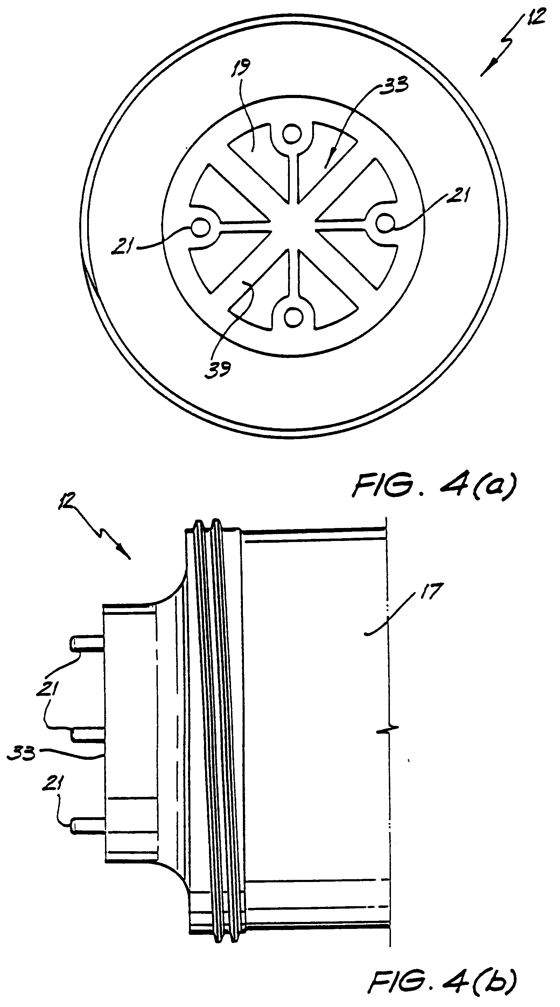

Turning particularly to Fig. 4(a), diaphragm valve support structure 12 is shown comprising a grid 33 having passages 19 extending therethrough. Projecting normal to grid 33 are four locating pins 21 which are adapted to extend through holes 30 as depicted in Fig. 3 through diaphragm valve 11.

Referring to Fig. 3, diaphragm valve 11 being formed of flexible material is provided with a pair of cross slits 29, each cross slit 29 corresponds with a grid bridge element 39.

Returning now to Fig. 1, which shows two separate operational positions of valve 11, each position being shown on opposite sides of centre line 41, diaphragm valve support structure 12 is positioned within housing 23 in lateral spaced relation thereto so as to provide an annular exit passage 27 therebetween. It should be appreciated that central portion 32 of diaphragm valve 11 is located within the major flow passage 19 whereas outer peripheral edge portion 31 of diaphragm valve 11 is located within exit passage 27.

So as to prevent diaphragm valve 11 from lifting away from grid 33, a plurality of positioning pins or projections 20 are moulded integrally with housing 23. Each positioning pin 20 is separated from diaphragm valve 28 by a thin gap 34. This gap should typically be as small as possible so as to prevent movement of diaphragm valve 11 without actually asserting force thereto in a rest position. Such force might otherwise distort valve 11.

The outer periphery 31 of diaphragm valve 11 is adapted to rest against an annular sealing ledge 28 of housing 23.

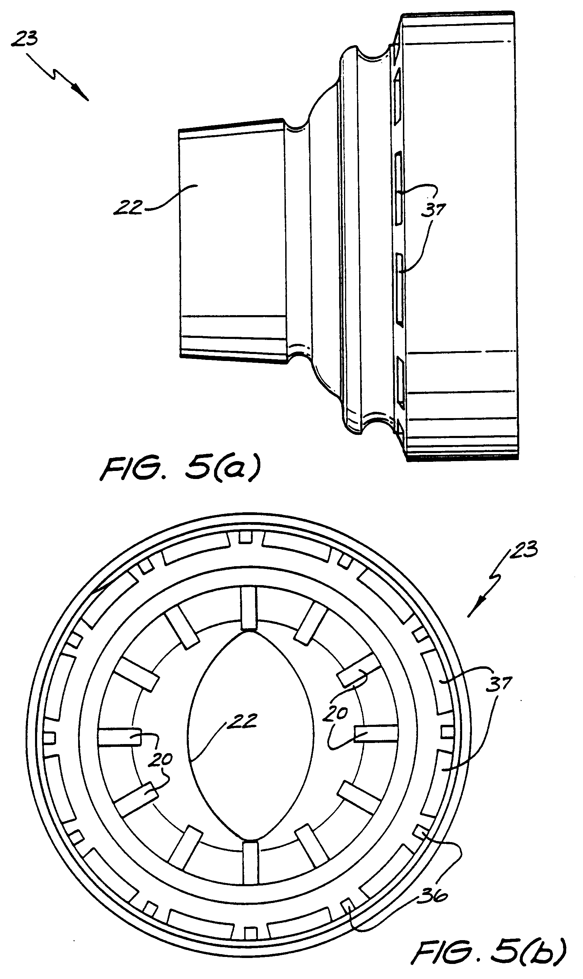

Housing 23 as best depicted in Fig. 5(a) shows a plurality of exit ports 37 spaced circumferentially therearound. Exit ports 37 are in fluid communication with exit passage 27 and allow exit of fluid from passage 27 to atmosphere.

Depicted in Fig. 5(b) is a number of protrusions 36 which serve to aid in retaining housing 23 to cylindrical shell 17.

In use, a standard inhaler mouthpiece will be inserted through aperture 18 so as to provide aerosol to chamber 15. A user would have placed his or her mouth over mouthpiece 22 or alternatively, the mask 13 which comprises nose cover 38 may be utilised to establish a fluid seal between aerosol holding chamber 15 and the user.

Upon breathing inwardly, central portion 32 of diaphragm valve 11 will open whilst at the same time outer edge portion 31 of diaphragm valve 11 will close onto sealing ledge 28. Accordingly, aerosol suspended within chamber 15 will pass through the diaphragm valve 11 to be breathed in by the user.

Upon the user exhaling, central portion 32 will close and be retained in a closed position against grid 33 of diaphragm valve support structure 12. At the same time, peripheral outer edge portion 31 of diaphragm valve 11 will open so as to allow the user's exhaled breath to escape via passage 27 and exit ports 37.

If necessary, several breaths may be taken wherein the operation of diaphragm valve 11 repeats the above described steps.

During an inward breath, non-return valves 40 will open allowing air to enter chamber 15 thus displacing the suspended aerosol therein. Upon an outward breath, non-return valves 40 will close so as to prevent any inadvertent escape of aerosol from chamber 15.

It should be appreciated that modifications and alterations obvious to those skilled in the art are not to be considered as beyond the scope of the present invention. For example, diaphragm valve support structure 12, rather than being glued or otherwise affixed to shell 17 may be moulded integrally therewith. Furthermore, the specific means of retaining diaphragm valve 11 to support structure 12 may be altered without departing from the scope of the invention.

In addition, a breathing indicator for example in the form of a thin section of mask 13 may be provided. As such, the thin section will protrude and retract to indicate breathing to a doctor for example.

The applicant also envisages that any suitable type of valve or valves may replace non-return valves 40.

## Claims

1. Apparatus comprising:
a housing (23) to co-operate with, attached to, or formed integrally with a chamber (15) to define a major fluid passage comprising an upstream zone within the chamber (15) and a downstream zone;
a major fluid exit (14) provided in the downstream zone;
a back-flow fluid exit (27) comprising an annular passage surrounding said major fluid passage; and
a diaphragm (11) mounted within the housing (23) and comprising a central region (32) comprising at least two slits (29) defining at least one flap interfacing the upstream and downstream zones and being openable to allow fluid flow therethrough from the upstream zone to the downstream zone and through the major fluid exit (14);
the apparatus being characterised by:
said annular passage comprising an annular sealing ledge (28); and
said diaphragm (11) further comprising a continuous peripheral region (31) across the back-flow fluid exit (27) which is in selective fluid communication with the downstream zone, is closable by said peripheral region (31) resting against said sealing ledge (28) when the central region (32) is opened, and is openable upon deflection of the peripheral region (31) away from said sealing ledge (28) when the central region (32) is closed and when fluid pressure in the downstream zone is higher than ambient pressure so as to allow escape of fluid from the downstream zone to atmosphere via the back-flow fluid exit (27).

2. In combination, the apparatus of claim 1 and the chamber (15), the chamber being characterised by a grid (33) extending across the major fluid passage adjacent to and supporting the diaphragm (11), the grid comprising passages (19) extending therethrough.

3. The combination of claim 2 further characterised by a plurality of locating pins (21) extending essentially normally from the grid (33), and wherein the diaphragm (11) is provided with a plurality of apertures (30) adapted to fit over said pins (21).

4. The apparatus of claim 1 characterised in that said central region (32) comprises at least two crossed slits (29) providing at least four flaps in said diaphragm (11).

5. The combination of claim 2 wherein said grid comprises at least one bridge (39) corresponding in location to the location of one of said slits in said diaphragm (11).

6. The combination of claim 2 further characterised by a flexible cap (16) fitted over one end of the housing (15) and comprising an aperture (18) through which a standard inhaler mouthpiece may be sealingly inserted so as to dispense airborne medication into the chamber (15).

7. The combination of claim 6 wherein said cap (16) is further characterised by a valve or constriction (40) adapted to separate the chamber (15) from the exterior thereof so as to impair back-flow of fluid from within the chamber (15) to atmosphere.

## Patentansprüche

1. Vorrichtung, die folgendes einbezieht:
Ein Gehäuse (23) für Zusammenarbeit mit einer angebrachten oder Integral geformten Kammer (15), um einen hauptsächlichen Flüssigkeitsdurchgang zu definieren, der eine Stromaufwärtszone innerhalb der Kammer (15) und eine Stromabwärtszone enthält;
ein hauptsächlicher Flüssigkeitsausgang (14), der in der Stromabwärtszone vorgesehen ist;
einen Rückströmausgang (27), der einen ringförmigen Durchgang einbezieht, der den besagten hauptsächlichen Flüssigkeits-durchgang umgibt; und
eine Membrane (11), die innerhalb des Gehäuses (23) montiert ist und eine zentrale Region (32)aufweist, die mindestens zwei Schlitze (29) enthält, welche mindestens eine Klappe definiert, die eine Schnittstelle zwischen den stromaufwärts und stromabwärts gelegenen Zonen bildet und die geöffnet werden kann, um eine Flüssigkeitsströmung ab der Stromaufwärtszone zur Stromabwärtszone durch diese und durch den Hauptflüssigkeitsausgang (14) fließen zu lassen;
die Vorrichtung wird durch folgendes gekennzeichnet:
besagten ringförmigen Durchgang, der eine ringförmige Dichtleiste (28) aufweist; und
besagte Membrane (11), die außerdem eine kontinuierliche periphere Region (31) quer über dem Rückströmflüssigkeitsausgang (27) aufweist, die mit der Stromabwärtszone in selektiver Flüssigkeitskommunikation steht, ist durch besagte periphere Region (31) verschließbar, die gegen besagte Dichtleiste (28) anliegt, wenn die zentrale Region (32) geöffnet wird, und kann nach Ablenkung der peripheren Region (31) weg von der besagten Dichtleiste (28) geöffnet werden, wenn die zentrale Region (32) geschlossen ist und, wenn der Flüssigkeitsdruck in der Stromabwärtszone höher ist als Umgebungsdruck, um das Entweichen von Flüssigkeit aus der Stromabwärtszone über den Rückströmflüssigkeitsausgang (27) in die Atmosphäre zu gestatten.

2. In Kombination, die Vorrichtung von Anspruch 1 und die Kammer (15), wobei die Kammer durch ein Gitter (33) gekennzeichnet ist, das sich quer über den Hauptflüssigkeitsdurchgang erstreckt, der neben der Membrane (11) liegt und diese unterstützt, wobei das Gitter hindurchgehende Passagen (19) aufweist.

3. Die Kombination von Anspruch 2 wird außerdem durch eine Vielzahl von Fixierstiften (21) gekennzeichnet, die sich normalerweise im wesentlichen ab dem Gitter (33) erstrecken, und worin die Membrane (11) mit einer Vielzahl von Öffnungen (30) versehen ist, die angepaßt sind um über die besagten Stifte (21) zu passen.

4. Die Vorrichtung des Anspruchs 1, dadurch gekennzeichnet, daß die besagte Region (32) mindestens zwei Kreuzschlitze (29) aufweist, die für mindestens vier Klappen in der besagten Membrane (11) sorgen.

5. Die Kombination des Anspruchs 2, worin das besagte Gitter mindestens eine Brücke (39) aufweist, die der Lage nach der Lage eines der besagten Schlitze in der besagten Membrane (11) entspricht.

6. Die Kombination des Anspruchs 2 ist außerdem durch eine flexible Kappe (16) gekennzeichnet, die über einem Ende des Gehäuses (23) angebracht ist und eine Öffnung (18) aufweist durch die ein normales Mundstück eines Inhalationsapparates abdichtend eingeschoben werden kann, um luftgetragene Medikation in die Kammer (15)auszugeben.

7. Die Kombination von Anspruch 6, worin die besagte Kappe (16) außerdem durch ein Ventil oder Verengung (40) gekennzeichnet ist, das bzw. die angepaßt ist die Kammer (15) von deren Außenseite zu trennen, um so die Rückströmung von Flüssigkeit ab dem Innenraum der Kammer (15) in die Atmosphäre zu beeinträchtigen.

## Revendications

1. Un appareil comprenant :
Un logement (23) fonctionnant avec une chambre (15), ou étant fixé à une chambre ou formant partie intégrale d'une chambre, et définissant une voie de passage principale pour fluides comportant une zone en amont à l'intérieur de la chambre (15) et une zone en aval;
une sortie principale pour fluides (14) située dans la zone en aval;
une sortie de reflux pour fluides (27) comportant une voie de passage annulaire entourant ladite voie de passage principale; et
un diaphragme (11) monté à l'intérieur du logement (23) et comprenant une partie centrale (32) comportant au moins deux fentes (29) définissant au moins un volet servant d'interface entre la zone en amont et la zone en aval et pouvant s'ouvrir pour laisser passer un fluide depuis la zone en amont vers la zone en aval à travers la sortie principale (14);
l'appareil étant caractérisé par :
le fait que ladite voie de passage annulaire comporte un rebord d'étanchéité annulaire (28); et
le fait que ledit diaphragme (11) comporte en outre une partie périphérique continue (31) montée en travers de la sortie de reflux (27), la partie périphérique continue (31) permettant une communication sélective des fluides avec la zone en aval, étant fermée lorsque ladite région périphérique (31) repose contre ledit rebord d'étanchéité (28) lorsque la partie centrale (32) est ouverte, pouvant être ouverte lorsque l'on écarte la partie périphérique (31) dudit rebord d'étanchéité (28) lorsque la partie centrale (32) est fermée et lorsque la pression des fluides contenus dans la zone en aval est supérieure à la pression ambiante afin de permettre l'échappement des fluides depuis la zone en aval vers l'atmosphère extérieure par l'intermédiaire de la sortie de reflux (27).

2. Une combinaison du dispositif de la revendication 1 et de la chambre (15), la chambre étant caractérisée par la présence d'une grille (33) montée en travers de la voie de passage majeure adjacente au diaphragme (11) et soutenant ce dernier, la grille comportant des voies de passage (19) passant à travers ce dernier.

3. La combinaison de la revendication 2 caractérisée en outre par la présence de plusieurs goupilles de positionnement (21) se prolongeant depuis la grille (33), et dans laquelle le diaphragme (11) est muni de plusieurs ouvertures (30) adaptées pour être ajustées sur lesdites goupilles (21).

4. Le dispositif de la revendication 1 caractérisé par le fait que ladite partie centrale (32) comprend au moins deux fentes croisées (29) formant au moins quatre volets dans ledit diaphragme (11).

5. La combinaison de la revendication 2 dans lequelle ladite grille comporte au moins un pont (39) dont l'emplacement correspond à l'emplacement de l'une desdites fentes dudit diaphragme (11).

6. La combinaison de la revendication 2 caractérisée en outre par la présence d'un couvercle flexible (16) monté sur l'une des extrémités du logement (23) et comportant une ouverture (18) pouvant servir à accueillir et à sceller une embouchure conventionnelle d'inhalateur afin de pouvoir diffuser des médicaments dissous dans l'air à l'intérieur de la chambre (15).

7. La combinaison de la revendication 6 dans lequel ledit couvercle (16) se caractérise en outre par la présence d'une valve ou d'une constriction (40) conçue pour séparer la chambre de l'extérieur afin d'empêcher la diffusion d'un reflux de fluides de l'intérieur de la chambre (15) vers l'atmosphère extérieure.
